(19) Europäisches Patentamt
European Patent Office
Office européen des brevets

(11) Veröffentlichungsnummer: **0 130 550**
**A2**

(12) # EUROPÄISCHE PATENTANMELDUNG

(21) Anmeldenummer: **84107388.5**

(22) Anmeldetag: **27.06.84**

(51) Int. Cl.⁴: **A 61 K 31/725**, A 61 K 9/72, A 61 K 47/00

(30) Priorität: **29.06.83 DE 3323389**

(43) Veröffentlichungstag der Anmeldung: **09.01.85 Patentblatt 85/2**

(84) Benannte Vertragsstaaten: **AT BE CH DE FR GB IT LI LU NL SE**

(71) Anmelder: **Intermedicat GmbH, Gerliswilstrasse 45, CH-6020 Emmenbrücke (CH)**

(72) Erfinder: **Breitenstein, Lilly, Dr., Desseler Strasse 1, D-3436 Hess.Lichtenau (DE)**
Erfinder: **Nehne, Jörg, Dr., Weserring 7, D-3501 Guxhagen (DE)**
Erfinder: **Feller, Wolfgang, Dr., Ernstbergstrasse 30, D-3508 Melsungen (DE)**

(74) Vertreter: **von Kreisler, Alek, Dipl.-Chem. et al, Deichmannhaus am Hauptbahnhof, D-5000 Köln 1 (DE)**

(54) **Auf Schleimhäute des Mundes, der Nase und/oder des Rachens wirkende Arzneimittel auf der Basis von Heparin und Tensiden.**

(57) Beschrieben werden auf die Schleimhaut des Mundes, der Nase und/oder des Rachens wirkende Arzneimittel, die Heparin und physiologisch verträgliche Tenside enthalten.

EP 0 130 550 A2

VON KREISLER SCHÖNWALD EISHOLD FUES
VON KREISLER KELLER SELTING WERNER



PATENTANWÄLTE
Dr.-Ing. von Kreisler † 1973
Dr.-Ing. K. Schönwald, Köln
Dr.-Ing. K. W. Eishold, Bad Soden
Dr. J. F. Fues, Köln
Dipl.-Chem. Alek von Kreisler, Köln
Dipl.-Chem. Carola Keller, Köln
Dipl.-Ing. G. Selting, Köln
Dr. H.-K. Werner, Köln

DEICHMANNHAUS AM HAUPTBAHNHOF
D-5000 KÖLN 1

B. Braun Melsungen AG
Melsungen

AvK/m 28. Juni 1983

Auf Schleimhäute des Mundes, der Nase und/oder des Rachens wirkende Arzneimittel auf der Basis von Heparin und Tensiden

Die Erfindung betrifft auf die Schleimhäute des Mundes, der Nase und/oder des Rachens wirkende Arzneimittel, die Heparin, Heparin-ähnliche Substanzen (Glykosaminoglykane), synthetisch oder halbsynthetisch hergestellte Heparinoide und Tenside enthalten.

Handelsübliches Heparin, das seit langen Jahren in der klinischen Anwendung im prophylaktischen und therapeutischen Bereich eingesetzte gerinnungshemmende und antithrombotisch wirkende Glykosaminoglykan, wird Patienten überwiegend als Alkali- oder Erdalkalisalz, vornehmlich in der Natrium- oder Calciumform, intravenös oder subkutan injiziert. Abgesehen von der kutanen/transkutanen äußerlichen Behandlung wurden verschiedentlich Versuche unternommen, alternative Routen der Heparinapplikation zu erproben.

Ein Gesichtspunkt zur Durchführung solcher Untersuchungen war, die systemische antikoagulatorische und antithrombotische Heparinwirkung, die Halbwertszeit im Plasma sowie möglicherweise auch die Häufigkeit von Nebenwirkungen wie Blutungen oder Thrombozytopenie unter gleichzeitiger Beibehaltung der hohen Sicherheit der Behandlung zu beeinflussen.

Einige wichtige Parameter, die im Rahmen solcher großangelegter Untersuchungen variiert wurden, sind:

Herstellung verschiedener organischer Salze des Heparins durch Ersatz der üblicherweise in Heparinlösungen vorliegenden Natrium- oder Calciumform.
Veränderung der Molekulargewichtsverteilung des handelsüblichen Heparins durch chemische Spaltung oder Fraktionierung nach verschiedenen Verfahrensweisen.
Veränderung der Ladungsdichte des eingesetzten Heparins.
Einsatz von Heparin, das mittels affinitätschromatographischer Verfahren, z.B. an Antithrombin III isoliert wird.
Kombination von Heparin und synergistisch wirkenden Substanzen wie Dihydroergotaminsalzen.
Ersatz des Heparins durch Heparinoide, die entweder in ihrer chemischen Struktur oder in ihrer Wirkung in vivo Heparinähnlichkeit aufweisen.
Variation des Applikationsortes und der Applikationsart der Heparin-haltigen Arzneimittel.

Untersuchungen, die subkutane oder intravenöse Applikation abzulösen, sind sowohl im tierexperimentellen

Stadium als auch bereits am Menschen in vielfacher Weise bekannt geworden.

Ziel dieser Arbeiten ist insbesondere, durch eine einfach durchzuführende Applikation bei möglichst geringem Substanzbedarf über lange Zeit einen konstant hohen oder niedrigen Wirkstoffspiegel im Blutplasma aufrecht zu erhalten. Für die Halbwertszeit von Heparin im Blut sind dagegen nur Werte von 1 - 2 Stunden gemessen worden.

Besondere Aufmerksamkeit ist Versuchen geschenkt worden, Heparin pulmonal zu verabreichen. Hierbei werden Heparinlösungen in fein verteilter Form als Aerosol in sehr hoher Dosierung intrapulmonal appliziert (DE-OS 28 01 867). Die Heparinwirkung hielt mehr als 24 Stunden an.

Übersichten und neuere Ergebnisse dieser Untersuchungen sind beispielsweise in folgenden Artikeln publiziert:

J. MAHADOO, J.G. HINES; L.B. JAQUES
Effect of Long-term Treatment of Mice with intrapulmonary Heparin.
Arzneimittel-Forsch./Drug Res. 31 (I), Nr. 6 (1981);

M. HELLGREN, K. HÄGNEVIK, M. BLOMBACK
Heparin Aerosol - Effects on Blood Coagulation and Pulmonary Function.
Thrombos. Res. 21, 493-502 (1981)

Die Aufnahme des Heparinwirkstoffs erfolgt in den genannten Versuchen durch die Tracheobronchialschleimhäute. Nachteilig bei dieser Behandlung ist unter anderem der recht hohe technische Aufwand der Applikation sowie vor allem der sehr hohe Heparinbedarf, der bei 5000 - 100 000 I.E. pro Verabreichung liegt. Das Behandlungsverfahren hat sich deshalb in der medizinischen Praxis nicht durchsetzen können.

Neben dieser seit mehreren Jahren untersuchten Methode gab es zahlreiche weitere Versuche, nach Heparin-haltigen Arzneimittelzubereitungen zu suchen, die einfach zu applizieren sind und gleichzeitig zu lang anhaltenden Wirkspiegeln im Blut führen. Zur Aufnahme von Heparin über die Darmschleimhaut wurde Widersprüchliches publiziert. Auch hier hat sich bisher kein marktreifes Präparat herstellen lassen.

L. STANZANI et al.
Rectal absorption of some glycosaminoglycansulphates and heparin in rats.
J. Pharm. Pharmacol 33, 773-786 (1981)

Ebenso sind einzelne Experimente, Heparin in Liposomen einzuschließen und zu applizieren, beschrieben worden. Ferner ist allerdings auch erwiesen, daß Heparin im Gastrointestinaltrakt (Magen) inaktiviert wird, so daß sich die orale Gabe von Heparin, ohne daß gleichzeitig besondere Maßnahmen zur Aufnahme getroffen werden, verbietet. Einige Anwendungen sind in den folgenden Schriften beansprucht:

GB-PS 1 117 649
GB-PS 1 135 784
US-PS 3 574 832
US-PS 3 842 014

Ziel eigener Untersuchungen war, Heparin-haltige Zubereitungen zu finden, die geeignet sind, Heparin oder seine Derivate einfach zu applizieren, dabei sicher in der Anwendung zu sein und eine kontrollierte und einstellbare Wirkstoffkinetik aufzuweisen.

Bei der Überprüfung einer Vielzahl Heparin-haltiger Zubereitungen konnte überraschenderweise nun gefunden werden, daß sich bei der Applikation dieser Mittel auf Schleimhäute des Mund-Nasen-Rachenraumes, Wirkspiegel im Blut nachweisen lassen. Dabei sind Abhängigkeiten von der Zusammensetzung der Zubereitung, der Dosis und der Dauer der Applikation nachweisbar.

Insbesondere ist verwunderlich, daß man bisher an der an sich sehr einfachen Möglichkeit, die Schleimhäute des Mund-Nasen-Rachenraumes als Auftrageort des Wirkstoffs Heparin in entsprechender Zubereitungsform zu nutzen, völlig vorbeigegangen ist.

Im Zuge umfangreicher optimierender Untersuchungen wurde gefunden, daß die Heparinwirkung, gemessen in Form von Heparin-Spiegeln im Plasma mit dem chromogenen Substrat S 2222, von zahlreichen Faktoren abhängig ist. Folgende Parameter wurden systematisch variiert:

Viskosität der Arzneimittelzubereitung.
Die eingesetzte Heparinmenge.
Der pH-Wert.
Die Art des Wirkstoffs (Heparin, Heparinderivate, Heparinoide).
Das Molekulargewicht und die Ladungsdichte des Heparins oder seiner Derivate.

Der Zusatz gefäßaktiver Mittel wie Ephedrin, Aludrin$^{R}$, Alupent$^{R}$, Epinephrin (Adrenalin).

Die Anwesenheit oberflächenaktiver tensidartiger, physiologisch verträglicher Substanzen.

Die Oberfläche des Mittels am Wirkort.

Die Mittel werden in Form von Tropfen, Gel oder in Form von Aerosolen in den Mund-Nasen-Rachenraum eingebracht.

Zusatz von Hilfsstoffen, die durch einen Film/Gelbildung das Austrocknen der Schleimhaut verhindern.

Art und Gehalt von Konservierungsstoffen.

Alle Versuchszubereitungen wurden keimarm zubereitet und den Versuchstieren (Kaninchen) in Form von Tropfen, Gel oder als Aerosol (Spray) auf die Rachen-, Mund- bzw. Nasenschleimhaut verabreicht. Nach definierten Zeiten wurden die Heparinspiegel im Blut der Tiere ermittelt. Dazu wird den Tieren jeweils 0,5 ml Blut als Citratblut aus der Ohrvene entnommen und Heparin im Plasma mit dem Heparin-sensitiven Test nach A.N. TEIEN und U. ABILDGAARD Assay of heparin in Plasma using a chromogenic substrate. Thromb. Res. 8, 413 (1976) mit S. 2222 nachgewiesen.

Als Ergebnis ist festzustellen, daß sich die Wirkspiegel im Sinne von Dosis-Wirkungsbeziehungen weitgehend beeinflussen lassen. Entscheidende Bedeutung kommt folgenden Faktoren zu:

Dem Molekulargewicht des eingesetzten Heparins, der Art und Menge des zugesetzten Tensids und der Form, in der sich der Wirkstoff auf den Schleimhäuten des Mundes, der Nase und des Rachens verteilt (Tropfen, Aerosol, Tampon).

Dementsprechend betrifft die Erfindung auf die Schleimhäute des Mundes, der Nase und/oder des Rachens wirkende Arzneimittel, die Heparin und physiologisch verträgliche Tenisde enthalten.

Die erfindungsgemäßen Arzneimittel machen erstmalig die therapeutische Applikation von Heparin auf die Mund-, Rachen- oder Nasenschleimhaut möglich, wobei je nach Zusammensetzung eine mehr oder weniger lang anhaltende Wirkstoffliberation zu beobachten ist und relativ wenig Heparin eingesetzt werden muß.

Die in den Arzneimitteln gemäß der Erfindung enthaltenen physiologisch verträglichen Tenside werden aus der Gruppe der anionischen Tenside, der nicht-ionischen Tenside und der ampholytischen (Zwitterionischen) Tenside ausgewählt.

Als Tenside kommen beispielsweise in Frage:

1. Anionische Tenside

wie Seifen, beispielsweise Natriumpalminat, Natriumstearat, Natriumoleat, Triethanolaminstearat
Natriumsalze von Fettalkoholsulfaten wie Natriumlaurylsulfat (Duponol$^{R}$ C)
oder Texapon $^{R}$ K 12, Natriumcetylstrearylsulfat (Lanette$^{R}$ E)
Sulfonsuccinate (Aerosol $^{R}$ OT)

2. Nichtionische Tenside

2a. Partielle Fettsäureester, mehrwertiger Alkohol, z.B.

Glycerinmonostearat

partielle Fettsäureester des Sorbitans: Sorbitan-monolaureat Span$^R$ oder Arlacel$^R$ 20

Sorbitanmonostearat oder -monooleat, Sorbitansesquioleat (Span$^R$/ Arlacel$^R$ 60/Arlacel$^R$ 80, Arlacel$^R$ 83)

Ester von Polyethylenglykolsorbitan wie z.B. das Monolaureat (Tween $^R$ 20), das Monostearat (Tween$^R$ 60) und das Monooleat (Tween$^R$ 80)

2b. Polyhydroxyethylen-Fettalkoholether

Polyhydroxethylencetylstearylether (Cetomakrogol) wie Cremophor$^R$ 0, Emulgin$^R$ C 1000,

Polyhydroxyethylen (4) Laurylether (Brij$^R$ 30)

Polyhydroxyethylen (23) Laurylether (Brij$^R$ 35)

2c. Polyhydroxyethylen-Fettsäureester

Polyhydroxyethylen (8) Stearat (Myrj$^R$ Cremophor$^R$ AP)

Polyhydroxyethylen (40) Staert (Myrj$^R$ 52)

Polyhydroxylethylen (100) Steart (Myrj$^R$ 59)

2d. Ethylenoxid-Propylenoxid-Blockcopolmyere

wie Pluronic$^R$ F 68

2e. Ethoxylierte Triglyceride

polyethoxyliertes Rizinusöl (40) wie Cremophor$^R$ EL

polyethoxyliertes hydriertes Rizinusöl wie Cremophor[R] RH, Cremophor[R] RH 40,
Cremophor[R] RH 60
Polyethoxylierte Öle (Labrofils[R])

3. Ampholytische Tenisde

wie Betaine und Sulfobetaine

der allgemeinen Formel

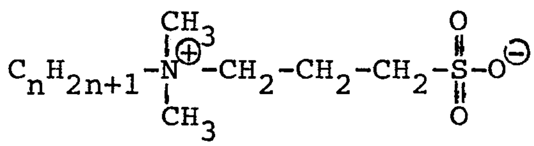

wobei n = 8 bis 18

Geeignete Tenside sind auch in dem bekannten Werk Hagers Handbuch der pharmazeutischen Praxis", Herausgeber P.H. List, L. Hörhammer, Springer-Verlag 1977 aufgeführt.

Die Arzneimittel gemäß der Erfindung enthalten das Heparin in Form seiner organischen Salze, seiner Alkali- oder Erdalkalisalze, insbesondere in der Natrium- oder Kalziumform. Anstelle des handelsüblichen Heparins können die Arzneimittel gemäß der Erfindung auch ein Heparin mit definiert kleinerem oder größerem mittlerem Molekulargewicht enthalten. Heparine unterschiedlicher Molekulargewichtsverteilung werden durch Ultrafiltration oder durch Gelfiltration über Sephadex[R] G-100 aus handelsüblichem Heparin erhalten. Anstelle des handelsüblichen Heparins oder damit zusammen oder anstelle eines Heparins mit definiert kleinerem oder größerem mittlerem Molekulargewicht oder damit zusammen können die erfindungsgemäßen Arzneimittel auch Heparinderivate oder Heparinoide enthalten. Heparinoide sind im Handel erhältlich, als Beispiele seien die Produkte SP 54 (Bene-Chemie, München) oder Arteparon[R] (Luitpold-Werk, München) genannt.

Die erfindungsgemäßen Arzneimittel liegen in Form von Lösungen, als Spray, als Gel, als Suspensionen oder in Form von getränkten Tampons vor und werden dementsprechend angewandt.

Als Dosierung kommen pro Applikation 5000 bis 100 000, vorzugsweise 1000 bis 10 000 I.E. Heparin oder entsprechend Heparin mit verringertem oder vergrößertem mittlerem Molekulargewicht oder entsprechend Heparinderivate oder Heparinoide in Frage.

Als Grundlage der erfindungsgemäßen Arzneimittel kann auf wässrige Lösungen, bevorzugt jedoch auf dem Fachmann bekannte Gelbildner, wie beispielsweise Polyethylenglykole, Polypropylenglykole, Polyethylen-Polypropylenmischpolymerisate, Methylcellulose, Carboxymethylcellulose, Gelatine, Alginate oder Polyacrylate zurückgegriffen werden, deren Art und Gehalt in weiten Grenzen nach Anforderung variabel ausgewählt werden kann.

Erfindungsgemäße Formulierungen der beanspruchten Arzneimittel mit Wirkung auf die Schleimhäute des Mundes, Rachens und der Nase sind Tropfen, Gele, Aerosole oder Nasentampons.

In diesem Sinne sind als Grundlage der Zubereitung lipophile Formen wie ölige Lösungen, ölige Sprays, Salbentampons, Nasensalben, Oleo-Gele, (Wasser-in-Öl) W/O-Salben und ölige Heparinsuspensionen anwendbar.

Als hydrophile Formen sind wässrige Lösungen wie (Nasen-)Tropfen, wässrige Sprays, Spüllösungen, Tampons, (Öl-in-Wasser) O/W-Emulsionen, Hydrogele und (Wasser-in-Öl) W/O-Salben anwendbar.

Aufgrund der Hydrophilie des Heparins werden vorzugsweise hydrophile Arzneimittelformen benutzt.

Den erfindungsgemäßen Arzneimitteln können auch übliche unbedenkliche Konservierungsmittel zugesetzt werden.

Ferner ist es möglich, den erfindungsgemäßen Arzneimitteln übliche bekannte Zusätze zum Ausgleich der Isotonie und des pH-Wertes zuzusetzen. Wenn die Zubereitungen isotonisch oder schwach hypertonisch sind, haben sie bevorzugt pH-Werte zwischen 5 und 8,0.

Die erfindungsgemäßen Arzneimittel können vorteilhaft sehr einfach appliziert werden und übertreffen damit jedes der bisher bekannten Verfahren zur Behandlung mit Heparin. Die Anwendung der erfindungsgemäßen Arzneimittel bietet deshalb praktische und therapeutische Vorteile.

Die erfindungsgemäßen Arzneimittel können insbesondere zur Verhinderung und Behandlung von tiefen Venenthrombosen, thromboembolischen Zwischenfällen, Arteriosklerose, Myocardinfarkt, akuten Arterienthrombosen, Embolien und Phlebitiden, Verbrauchskoagulopathie, schmerzhaften Haemorrhoidalknoten sowie systemischen Anwendungen in extrakorporalen Blutkreisläufen eingesetzt werden.

Der Gehalt an Tensiden in den erfindungsgemäßen Arzneimitteln liegt im Bereich vom 0,0001 Gew.-% bis 60 Gew.-%, vorzugsweise im Bereich von 0,1 bis 20 Gew.-%.

Die erfindungsgemäßen Arzneimittel können zusätzlich gefäßaktive Substanzen enthalten. Diese Substanzen sind dem Fachmann bekannt, als Beispiele seien genannt: Epinephrin (Adrenalin), Ephedrin, Aludrin$^R$ und Alupent$^R$.

Die erfindungsgemäßen Arzneimittel können leicht dadurch hergestellt werden, daß man im einfachsten Fall wässrige Heparinlösungen bekannten Gehaltes mit wässrigen Lösungen von Tensiden mischt, so daß durch geeignete Wahl der Konzentrationen und Mischungsverhältnisse unter Zusatz von die Viskosität beeinflussenden Hilfsstoffen, von Filmbildnern oder auch in Kombination mit gefäßerweiternden Mitteln Lösungen entstehen, die unmittelbar nach ihrer Herstellung oder nach Zusatz von Konservierungsstoffen auch nach Lagerung eingesetzt werden können. Dem Fachmann bekannte Bedingungen, wie die Unbedenklichkeit und Verträglichkeit der eingesetzten Hilfs- und Trägerstoffe und ihrer Mischungen oder die Auswahl der pH-Werte bedürfen keiner näheren Erläuterungen.

In tierexperimentellen Kontrollexperimenten erwies sich nach Applikation von Heparinlösungen auf die Mund-, Rachen- oder Nasenschleimhaut, daß auch in Abwesenheit von Tensiden in Einzelfällen Heparinspiegel im Plasma gemessen werden konnten.
Wie sich jedoch zeigte, sind die so ermittelten Heparinspiegel nicht reproduzierbar.
Außerdem konnte auf diese Weise keine kontrollierte Einstellung der Wirkstoffkinetik ereicht werden.

Der Einsatz des Heparins auf die Schleimhäute des Kopfes ohne erfindungsgemäßen Zusatz von Tensiden verbietet sich deshalb auch aus Gründen der Arzneimittelsicherheit.

Beispiel 1

4,5 g eines viskosen Gels enthalten:

| | |
|---|---|
| 0,202 g | Eudispert hv (Röhm Pharma GmbH) |
| 0,058 g | NaOH |
| 0,517 g | Glycerin |
| 0,800 g | Cetomakrogol 1000 (Croda Chemicals) Tensid |
| 0,155 g | Na-Heparinat ≙10 000 I.E./ml |
| 2,768 g | $H_2O$ |

Diese Grundmasse wurde dahingehend verändert, daß das mittlere Molekulargewicht des Heparins (Tabelle I), sowie Art und Menge des Tensids (hier Cetomakrogol) variiert wurde (Tabelle II).

Die Versuchszubereitungen wurden entweder in Tropfenform oder tampongetränkt auf die Nasenschleimhaut oder die Mund/Rachen-Schleimhaut narkotisierter Kaninchen aufgegeben und zeitabhängig die Heparinspiegel verfolgt. Das Ergebnis ist in Tabelle I und II in Form der gemessenen Heparinspiegel zusammengefaßt. Es wurden hier zu Versuchszwecken jeweils 1000 I.E./kg Körpergewicht des Versuchstieres Heparin appliziert.

Tabelle I

Zeitabhängige Heparinspiegel I.E./ml Plasma nach Applikation eines Gels auf die Nasenschleimhaut bzw. auf die Mund/Rachenschleimhaut.
(Abhängigkeit von der Art, dem Molgewicht und der Menge des eingesetzten Antithrombotikums)

| | Nasenschleimhaut | | Mund/Rachenschleimhaut | |
|---|---|---|---|---|
| | nach | | nach | |
| Zubereitung | 2 Std. | 20 Std. | 2 Std. | 20 Std. |
| Heparin, handelsüblich | 0,3 | 0,08 | 0,25 | 0,05 |
| Heparinoid SP 54$^{R}$ | 0,4 | 0,06 | 0,1 | 0 |
| Heparinoid | 0,6 | 0,04 | 0,3 | 0 |
| Heparin, Mol Gew 5000 | 0,5 | 0,15 | 0,4 | 0,8 |
| Heparin, Mol Gew 8400 | 0,45 | 0,16 | 0,35 | 0,07 |
| Heparin, Mol Gew 10300 | 0,4 | 0,07 | 0,09 | 0 |
| Heparin, Mol Gew 1600 | 0,1 | 0 | 0,08 | 0 |
| Heparin, handelsüblich 0,0775g/4,5kg | 0,2 | 0,06 | 0,2 | 0,05 |
| Heparin, handelsüblich 0,020g/4,5kg | 0,1 | 0,05 | 0,1 | 0 |

Tabelle II

Zeitabhängige Heparinspiegel IE/ml Plasma nach Applikation auf die Nasenschleimhaut durch Tropfen bzw. Tampons.

(Abhängigkeit von der Art und Menge des Tensids)

| Zubereitung | Tropfen | | Tampon | |
|---|---|---|---|---|
| | 2 Std. | 20 Std. | 2 Std. | 20 Std. |
| Grundmasse | 0,3 | 0,08 | 0,3 | 0,15 |
| Ersatz von Cetomakrogol durch folgende Tenside: | | | | |
| Span 80 | 0,2 | 0,09 | 0,2 | 0,13 |
| Pluromic$^R$ F68 | 0,4 | 0,10 | 0,45 | 0,19 |
| Na-Stearat | 0,1 | 0,06 | 0,18 | 0,09 |
| Glycerinmonostearat | 0,15 | 0 | 0,15 | 0 |
| Lecithin | 0,08 | 0 | 0,08 | 0 |
| Carbopol 940 | 0,19 | 0,12 | 0,21 | 0,13 |
| Cremophor$^R$ EL | 0,20 | 0,14 | 0,25 | 0,11 |
| Ersatz von 0,89 g Cetomakrogol durch | | | | |
| 0,4 g " | 0,3 | 0,07 | 0,35 | 0,12 |
| 0,2 g " | 0,22 | 0,06 | 0,38 | 0,15 |
| 0,1 g " | 0,1 | 0,1 | 0,1 | 0,1 |

Beispiel 2

Es wurde eine Zubereitung folgender Zusammensetzung hergestellt:

| | |
|---|---|
| PEG 1000 | 0,634 g |
| PEG 500 | 0,694 g |
| Cetomakrogol 1000 | 0,800 g |
| Heparin-Natrium als wässrige Lösung mit 1 ml ≙ 37 000 I.E. | 10 000 I.E. Heparin |

Auch diese Zubereitung wurde verändert, indem Art und Menge des Tensids durch die in Tabelle II angegebenen Tenside anstelle des Cetomakrogol 1000 ersetzt wurde. Auch hier konnten in jedem Fall in Anwesenheit eines Tensids meßbare Heparinspiegel gefunden werden.

Beispiel 3

Eine wässrige Lösung der folgenden Zusammensetzung wurde in Form von Tropfen oder eines Sprays auf die Schleimhäute der Nase oder in den Mund- bzw. Rachenraum von 3 freiwilligen gesunden Probanden appliziert. Die hier verwendete Dosis lag bei 10 000 I.E. pro Behandlung. Die Heparinspiegel im Plasma wurden ermittelt (Tabelle III).

5 ml Lösung enthalten:

660 ml Heparin-Natrium
0,2 g Cetomakrogol 1000

Tabelle III
Heparinspiegel I.E./ml in Plasma, gemessen nach Teien et al. loc. cit., mit S-2222

| | Proband 1 | | Proband 2 | | Proband 3 | |
|---|---|---|---|---|---|---|
| | nach | | nach | | nach | |
| | 2 Std. | 20 Std. | 2 Std. | 20 Std. | 2 Std. | 20 Std. |
| Nasentropfen | 0,25 | 0,1 | 0,15 | 0,08 | 0,20 | 0,15 |
| Nasenspray | 0,4 | 0,2 | 0,32 | 0,10 | 0,28 | 0,15 |
| Mundtropfen | 0,1 | 0 | 0 | 0 | 0,15 | 0 |
| Mund/Rachenspray | 0,15 | 0,1 | 0,10 | 0,1 | 0,20 | 0 |

## Patentansprüche

1. Auf die Schleimhäute des Mundes, der Nase und/ oder des Rachens wirkende Arzneimittel, enthaltend Heparin und physiologisch verträgliche Tenside sowie übliche Träger- und Hilfsstoffe.

2. Arzneimittel nach Anspruch 1, dadurch gekennzeichnet, daß die Tenside aus der Gruppe der anionischen, nicht-ionischen oder ampholytischen (zwitterionischen) Tenside ausgewählt sind.

3. Arzneimittel nach Ansprüchen 1 und 2, dadurch gekennzeichnet, daß der Gehalt der Tenside im Bereich von 0,0001 bis 60 Gew.-%, vorzugsweise im Bereich von 0,1 bis 20 Gew.-% liegt.

4. Arzneimittel nach Ansprüchen 1 bis 3, dadurch gekennzeichnet, daß sie Heparin in Form seiner organischen Salze, Alkali- oder Erdalkalisalze, insbesondere in der Natrium- oder Calciumform, enthalten.

5. Arzneimittel nach Ansprüchen 1 bis 4, dadurch gekennzeichnet, daß sie ein Heparin mit definiert kleinerem oder größerem mittlerem Molekulargewicht als handelsübliches Heparin enthalten.

6. Arzneimittel nach Ansprüchen 1 bis 5, dadurch gekennzeichnet, daß sie Heparinderivate und/oder Heparinoide enthalten.

7. Arzneimittel nach Ansprüchen 1 bis 6, dadurch gekennzeichnet, daß sie in Form von Lösungen, in Form von Sprays, in Form von Gelen, in Form von Suspensionen oder in Form von getränkten Tampons vorliegen.

8. Arzneimittel nach Ansprüchen 1 bis 7, dadurch gekennzeichnet, daß sie zusätzlich gefäßerweiternde Substanzen enthalten.